# EUROPEAN PATENT APPLICATION

(11) **EP 1 333 088 A1**
(43) Date of publication of application: **06.08.2003**
(21) Application number: 01970217.4
(22) Date of filing: 26.09.2001
(51) Int. Cl.: C12N 15/09, A61K 35/76, A61K 38/00, A61K 48/00, A61P 21/00, A61P 25/02

(54) **PARAMYXOVIRUS VECTORS FOR INTRODUCING FOREIGN GENES INTO SKELETAL MUSCLE**

(30) Priority: 06.10.2000 JP 2000308533
(71) Applicant: Dnavec Research Inc., Ibaraki 305-0856 (JP)
(72) Inventor: FUKUMURA, Masayuki, Tsukuba-shi, Ibaraki 305-0856 (JP); SHIOTANI, Akihiro, Tokyo 112-0006 (JP); MAEDA, Mitsuyo, Sakai-shi, Osaka 591-8035 (JP); HASEGAWA, Mamoru, Tukuba-shi, Ibaraki 305-0856 (JP)
(74) Representative: VOSSIUS & PARTNER
(86) International application number: JP0108372
(87) International publication number: WO02031138

(57) **Abstract**

Whether recombinant Sendai virus (SeV) vector can be used for transporting genes into skeletal muscle was examined using LacZ reporter gene and insulin-like growth factor gene. As a result, transgene expression continued at longest for 1 month after the injection. Compared with control, the transduction of the insulin-like growth factor gene caused a significant increase in regenerated fibers and splitting myofibers, i.e., an index of hypertrophy. Furthermore, the total number or myofibers increased by the gene. Thus, Paramyxovirus vectors, including Sendai virus, were shown to achieve a high-level expression of transgenes in skeletal muscle; and the high potential of the transduction of an insulin-like growth factor gene using a Paramyxovirus vector in treating neuromuscular disorders was indicated.

## Description

### Technical Field

The present invention relates to Paramyxovirus vectors for introducing foreign genes into skeletal muscle.

### Background Art

The subfamily *Pramyxovirinae* comprises three genera (*Rhabdoviridae, Paramyxoviridae*, and *Filoviridae)* of enveloped viruses that contain non-segment negative strand RNA genomes that function as templates for the synthesis of mRNA and anti-stranded genomes. Sendai virus (SeV) belongs to *Paramyxoviridae* whose pathogenicity to human has been denied from early studies in virology (Nagai, Y. and Ishihama, A., The viral experimental protocol (1995. 4)). SeV has a strict cytoplasmic life cycle in mammalian cells and its transferred RNA is maintained in the cytoplasm without interacting with the chromosomes of host cell. Therefore, SeV may be safely used for human gene therapy. The entire genome nucleotide sequence of the SeV Z strain used herein had been determined by Shibuta *et al*. in 1986. Success in the recovery of an infectious virus from the transfected cDNA of SeV enabled genetic engineering technology (Conzelmann, K. K. , Annu. Rev. Genet. 32, 123-162 (1998); Nagai, Y., and Kato, A., Microbiol. Immunol. 43, 613-624 (1999)). Moreover, a variety of foreign genes with additional transcription units have been inserted at appropriate genome positions and have been expressed in SeV at extremely high levels (Yu, D., Shioda, T., Kato, A., Hasan, M. K., Sakai, Y., and Nagai, Y., Genes Cells 2, 457-466 (1997)).

Insulin like growth factor I (IGF-I) plays an important role in the development, maintenance, and regeneration of skeletal muscle. The effect of IGF-I on myogenic cells includes stimulation of myoblast replication, myogenic differentiation, and myotube hypertrophy (Annu. Rev. Physiol. 53, 201-216 (1991); Endocr. Rev., 17 481-516 (1996); Cell. 75, 59-72 (1993); Genes Dev. 7, 2609-2617 (1993)). In muscle regeneration, proliferation of muscle precursor cells, fusion into myotubes and reinnervation are involved. IGF-I, which is produced in satellite cells, acts as a powerful stimulant for proliferation and differentiation of muscle precursor cells (Acta Physiol. Scand. 167, 301-305 (1999)). IGF-I gene transfer to skeletal muscle has already been applied to the treatment of denervated skeletal muscle atrophy using non-viral technology and age-related loss of skeletal muscle function using the AAV vector (Proc. Natl. Acad. Sci. USA. 95, 15603-15607 (1998)).

### Disclosure of the Invention

An objective of the present invention is to provide Paramyxovirus viral vector that ensures highly efficient transfer of foreign genes into skeletal muscle cells and use thereof. More specifically, the present invention provides a Paramyxovirus vector for introducing foreign genes into skeletal muscle, composition comprising the vector for foreign gene transfer into skeletal muscle, and method for introducing foreign genes into skeletal muscle using this vector. As a preferred embodiment of the present invention, a Paramyxovirus vector wherein a gene encoding a insulin-like growth factor has been inserted is provided, and use of this vector for the formation of myofibers are provided.

Skeletal muscle is an attractive site for the delivery and expression of exogenous genes encoding therapeutic proteins for the treatment of systemic diseases and neuromuscular disorders. The present inventors investigated the feasibility of using the recombinant Sendai virus (SeV) vector containing LacZ reporter gene and human insulin-like growth factor-I (hIGF-I) gene for gene delivery into skeletal muscle

A large number of X-gal labeled myofibers were detected in animals with/without bupivacaine treatment 7 days after the intramuscular injection of LacZ/SeV, and the transgene expression continued up to one month post-injection. Recombinant hIGF-I derived from IGF-I/SeV was detected as the major protein species in culture supernatants of L6 cells; and thus the induced L6 cells was determined to undergo morphological changes, such as multinuclear organization and hypertrophy. The introduction of IGF-I/SeV into the muscle led to significant increases in regenerating myofibers and splitting myofibers, which were indicative of hypertrophy, and also an increase in the total number of myofibers, in comparison to those seen in the LacZ/SeV treated control. These results demonstrated that Paramyxovirus containing SeV achieves high-level transgene expression in skeletal muscle, and that IGF-I gene transfer using Paramyxovirus vector may have a great potential in the treatment of neuromuscular disorders.

Specifically, the present invention relates to a Paramyxovirus vector gene for introducing a foreign gene into skeletal muscle, method for introducing a foreign gene into skeletal muscle using the vector, and a Paramyxovirus vector wherein an insulin-like growth factor has been inserted as a foreign gene, and use thereof for the formation of myofibers. More specifically, the present invention provides:
[1] a method for introducing a foreign gene into skeletal muscle, wherein said method comprises the step of administering a Paramyxovirus vector inserted with the foreign gene into skeletal muscle;
[2] the method according to [1], wherein the Paramyxovirus is Sendai virus;
[3] the method according to [1] or [2], wherein the foreign gene is a therapeutic gene;
[4] the method according to [1] or [2], wherein the foreign gene is a gene that encodes an insulin-like growth factor;
[5] a Paramyxovirus vector inserted with a foreign gene which is used for introducing the foreign gene into skeletal muscle;
[6] the vector according to [5], wherein the Paramyxovirus is Sendai virus;
[7] the vector according to [5] or [6], wherein the foreign gene is a therapeutic gene;
[8] the vector according to [5] or [6], wherein the foreign gene encodes an insulin-like growth factor;
[9] a composition for introducing a foreign gene into skeletal muscle which comprises a Paramyxovirus vector inserted with the foreign gene;
[10] the composition according to [9], wherein the Paramyxovirus is Sendai virus;
[11] the composition according to [9] or [10], wherein the foreign gene is a therapeutic gene;
[12] the composition according to [9] or [10], wherein the foreign gene encodes an insulin-like growth factor;
[13] the composition according to [12] which is used for increasing regenerating myofibers and/or splitting myofibers in mammal; and
[14] the composition according to [12] which is used for the treatment of neuromuscular disorders.

Herein, the phrase "Paramyxovirus vector" is defined as a vector (or carrier) that is derived from Paramyxovirus and that is used for gene transfer into host cells. The Paramyxovirus vector of the present invention may be ribonucleoprotein (RNP) or a virus particle having infectivity. Herein, "infectivity" is defined as the ability of the recombinant Paramyxovirus vector to transfer, through its cell adhesion and membrane fusion abilities, a gene contained in the vector into cells to which the vector is adhered. In a preferred embodiment, a foreign gene is integrated into the Paramyxovirus vector of the present invention in an expressible manner by genetic engineering. The Paramyxovirus vector may have replication ability, or may be a defective vector without replication ability. Herein, "replication ability" is defined as an ability of virus vectors to replicate and produce infective virus particles in host cells that are infected with the virus vectors.

Herein, the term "recombinant" Paramyxovirus vector is defined as a Paramyxovirus vector constructed via genetic engineering or amplified products thereof. For instance, recombinant Paramyxovirus vectors can be generated by reconstitution from a recombinant Paramyxovirus cDNA (Nagai, Y., and Kato, A., Microbiol. Immunol. 43, 613-624 (1999)).

Herein, the term "Paramyxovirus" is defined as a virus of the *Paramyxoviridae* family or a derivative thereof. Paramyxoviruses that can be used in the present invention include, viruses belonging to the family *Paramyxoviridae*, such as, Sendai virus, Newcastle disease virus, Mumps virus, Measles virus, respiratory syncytial virus (RSV), rinderpest virus, distemper virus, simian parainfluenza virus (SV5), and type I, II, and III human parainfluenza virus. The virus of the present invention may be preferably a virus of the genus Paramyxovirus or a derivative thereof.

Examples of Paramyxoviruses that can be used in the present invention include type 1 parainfluenza viruses, such as, Sendai virus and human HA2 virus; type 2 parainfluenza viruses, such as monkey SV5, SV41, and human CA virus; type 3 parainfluenza viruses, such as bovine SF and human HA1 virus; type 4 parainfluenza viruses (including subtypes A and B); Mumps virus; Newcastle disease virus; and numerous other Paramyxoviruses. Most preferably, the Paramyxovirus of the present invention may be the Sendai virus. These viruses may be naturally-occurring, mutants, laboratory-passaged strains, artificially constructed strains, etc. Incomplete viruses such as the DI particle (Willenbrink, W., and Neubert, W. J. , J. Virol. 68, 8413-8417 (1994)), synthesized oligonucleotides, and so on, may also be utilized as material for generating a viral vector of the present invention.

Genes encoding proteins of Paramyxovirus include NP, P, M, F, HN, and L genes. Herein, the "NP, P, M, F, HN, and L genes" represent those encoding the nucleocapsid protein, phosphoprotein, matrix protein, fusion protein, hemagglutinin-neuraminidase, and large protein, respectively. Genes of each virus of the subfamily Paramyxovirus are described generally as follows. In general, NP gene may also be indicated as "N gene."

| | | | | | | | |
|---|---|---|---|---|---|---|---|
| Paramyxovirus | NP | P/C/V | M | F | HN | - | L |
| Rublavirus | NP | P/V | M | F | HN | (SK) | L |
| Morbillivirus | NP | P/C/V | M | F | H | - | L |

For instance, the accession numbers of each gene of Sendai virus classified as a Respirovirus of *Paramyxoviridae* in the nucleotide sequence database, are M29343, M30202, M30203, M30204, M51331, M55565, M69046, and X17218 for NP gene; M30202, M30203, M30204, M55565, M69046, X00583, X17007, and X17008 for P gene; D11446, K02742, M30202, M30203, M30204, M69046, U31956, X00584, X53056 for M gene; D00152, D11446, D17334, D17335, M30202, M30203, M30204, M69046, X00152, and X02131 for F gene; D26475, M12397, M30202, M30203, M30204, M69046, X00586, X02808, X56131 for HN gene; and D00053, M30202, M30203, M30204, M69040, X00587, and X58886 for L gene.

As used herein, the term "gene" refers to a genetic substance including nucleic acids, such as RNA and DNA. A gene may or may not encode a protein. For example, a gene may encode a functional RNA such as ribozyme or antisense RNA. A gene can be a naturally-occurring sequence or an artificially designed sequence. Furthermore, as used herein, the term "DNA" includes single-stranded DNA and/or double-stranded DNA.

Herein, the term "skeletal muscle," as employed in this specification refers to muscles showing a striped pattern, and includes cardiac muscle.

The present invention provides the use of Paramyxovirus vector for gene transfer into skeletal muscle. The present inventors found that Sev of Paramyxovirus could allow introduction of genes into skeletal muscle with high efficiency. Skeletal muscle is important as a target for the treatment of systemic diseases and neuromuscular disorders , and thus the vector of the present invention can be suitably used for gene therapy of such diseases.

In addition, the present inventors revealed that the genes transferred into skeletal muscle using a recombinant SeV vector were persistently expressed over one month. This shows an advantage that continuous therapeutic effect can be obtained when gene therapies targeted at skeletal muscle was performed using a recombinant SeV vector.

It is suggested that Paramyxovirus vectors can be preferably utilized in clinical trials of human gene therapy because they are safe and also because the vectors are not pathogenic to humans . First, it is a major obstacle in high efficient gene expression that introduced DNA must be transported into the nucleus, or the nuclear membrane must be lost for the expression of a foreign gene. However, in the case of Sendai virus and such expression of a foreign gene in host cell is driven by both cellular tubulin and its own RNA polymerase (L protein) in the cytoplasm with the replication of viral genome. This suggests that the genome of Sendai virus does not interact with the chromosome of host cells, which means that chromosomal aberration and immortalization leading to risks such as tumorigenesis are avoided. Second, the Sendai virus is known to be pathogenic in rodents causing pneumonia, but not in humans, which is supported by studies showing that the intranasal administration of wild type Sendai virus does not harm nonhuman primates (Hurwitz, J. L. et al., Vaccine 15, 533-540 (1997)). These features suggest that Sendai virus vector can be utilized in human therapy, and further support the notion that Sendai virus can be a promising alternative in gene therapy to skeletal muscle.

Thus, the finding of the present inventors that Paramyxovirus vector is effective in gene transfer into skeletal muscle may greatly advance gene therapy, especially those targeted at skeletal muscle.

The recombinant Paramyxovirus vector of the present invention used for gene transfer into skeletal muscle is not limited to any specific kind. For instance, suitable Paramyxovirus vectors include vectors that are able to replicate and autonomously propagate. In general, the genome of wild type Paramyxovirus contains a short 3' leader region followed by six genes encoding N (nucleocapsid), P (phospho), M (matrix), F (fusion), HN (hemagglutinin-neuraminidase), and L (large) proteins, and has a short 5' trailer region at the other terminus. The vector of the present invention that is able to replicate autonomously can be obtained by designing a genome having a similar structure to that described above. In addition, a vector for expressing an exogenous gene can be obtained by inserting the exogenous gene to the genome of the above vector. The Paramyxovirus vector of the invention may have an altered alignment of virus genes compared to the wild type virus.

The Paramyxovirus vector of the invention may have deletion(s) of some of the genes that are contained in wild type virus. For instance, when Sendai virus vector is reconstituted, proteins encoded by NP, P/C, and L genes are thought to be required in trans, but the genes are not required to be a component of the virus vector. In one embodiment, an expression vector carrying genes encoding the proteins may be co-transfected into host cells with another expression vector encoding the vector genome to reconstitute a virus vector. Alternatively, an expression vector encoding the virus genome is transfected into host cells carrying genes encoding the proteins, and thus a virus vector can be reconstituted using the proteins provided by the host cell. The amino acid sequence of these proteins are not required to be identical to those derived from the original virus as long as they have an equivalent or higher activity in nucleic acid transfer, and may be mutated or replaced with those of homologous genes of other viruses.

Proteins encoded by M, F, and HN genes are thought to be essential for cell-to-cell propagation of a Paramyxovirus vector. However, these proteins are not required when the vector is prepared as RNP. If genes M, F, and HN are components of the genome contained in RNP, products of these genes are produced when introduced into host cells, and virus particles having infectivity are generated.

RNP can be introduced into cells as a complex with a transfection reagent, such as, lipofectamine and polycationic liposome. Specifically, a variety of transfection reagents can be used, for instance, DOTMA (Boehringer), Superfect (QIAGEN #301305), DOTAP, DOPE, DOSPER (Boehringer #1811169), etc. Chloroquine may be added to prevent degradation in the endosome (Calos, M. P., Proc. Natl. Acad. Sci. USA 80, 3015 (1983)). In the case of replicative viruses, the produced viruses can be amplified or passaged by re-infecting them into cultured cells, chicken eggs, or animals (e.g., mammals such as mice).

Vectors lacking the M, F, and/or HN genes are also used as the Paramyxovirus vector of the present invention. These vectors can be reconstituted by exogenously providing deleted gene products. Such vectors can still adhere to host cells and induce cell fusion like the wild type. However, daughter virus particles do not have the same infectivity as the original ones because the vector genome introduced into cells lacks one of the above genes. Therefore, these vectors are useful as safe virus vectors that are capable of only a single gene transfer. For instance, genes deleted from the genome may be F and/or HN genes. Virus vectors can be reconstituted by co-transfection of an expression plasmid encoding the genome of a recombinant Paramyxovirus lacking the F gene, an expression vector for the F protein, and that for NP, P/C, and L proteins into host cells (International Application numbers PCT/JP00/03194 and PCT/JP00/03195). Alternatively, host cells wherein the F gene is integrated into the chromosome may also be used for the reconstitution of vectors. The amino acid sequences of these exogenously provided proteins do not have to be identical to those of the wild type and may be mutated or replaced by homologous proteins of other viruses as long as they provide equivalent or higher gene transfer activity.

The envelope protein of the Paramyxovirus vector of the invention may contain a protein other than the envelope protein of the original vector genome. According to the present invention, there is no limitation on such proteins. These include envelope proteins of other viruses such as the G protein of the vesicular stomatitis virus (VSV-G). Thus, the Paramyxovirus vector of the invention includes a pseudo type virus vector that has an envelope protein derived from a virus different from the original virus.

The Paramyxoviral vector of the present invention may also comprise, for example, on the viral envelop surface, proteins capable of adhering to particular cells, such as adhesion factors, ligands and receptors, or chimeric proteins that comprises such proteins on the outer surface and viral envelop-derived polypeptides inside the virus. Such proteins enable production of a vector targeting a particular tissue. These proteins may be encoded by the virus genome itself, or supplied at the time of virus reconstitution through the expression of genes other than from the virus genome (for example, genes derived from another expression vector or host cell chromosome) .

The virus genes contained in the vector of the present invention may be altered, for example, to reduce antigenicity or enhance RNA transcription efficiency or replication efficiency. Specifically, it is possible to alter at least one of the NP, P/C, and L genes, which are genes of replication factors, to enhance transcription or replication. It is also possible to alter the HN protein, a structural protein having hemagglutinin activity and neuraminidase activity, to enhance the virus stability in blood by weakening the former activity and to regulate infectivity by altering the latter activity. The fusion ability of membrane-fused liposomes can be altered by modifying the F protein that is involved in membrane fusion. Furthermore, it is possible to analyze the antigen presenting epitopes and such of possible antigenic molecules on the cell surface, such as the F protein and HN protein, and use them to generate a Paramyxovirus that is engineered to have weak antigen presenting ability.

A viral vector of the present invention can encode foreign genes in its genomic RNA. A recombinant Paramyxovirus vector comprising foreign genes can be prepared by inserting foreign genes into the above-mentioned Paramyxovirus vector genome. The foreign gene can be a desired gene to be expressed in target skeletal muscle. The foreign gene may encode a naturally occurring protein, or a modified. protein prepared by modifying the original protein by deletion, substitution or insertion, as long as the modified protein is functionally equivalent to the naturally occurring protein. For instance, for the purpose of gene therapy and such, a gene to treat a target disease may be inserted into the virus vector DNA. In the case of inserting a foreign gene into Sendai virus vector DNA, a sequence having nucleotides of multiples of six is desirably inserted between the transcription end sequence (E) and the transcription start sequence (S) (Calain, P., and Roux, L., J. Virol. 67(8), 4822-4830 (1993)). A foreign gene can be inserted upstream and/or downstream of each of the virus genes (NP, P, M, F, HN, and L genes). In order not to interfere with the expression of upstream and downstream genes, an E-I-S sequence (transcription end sequence-intervening sequence-transcription start sequence) or a portion of it may be suitably placed upstream or downstream of the foreign gene. Alternatively, the foreign gene can be inserted via IRES sequence.

Expression level of inserted foreign genes can be regulated by the type of transcription start sequence that is attached to the upstream of the genes. It also can be regulated by the position of insertion or the sequence surrounding the gene. In Sendai virus, for instance, the closer the insertion position to the 3'-terminus of the negative strand RNA of the virus genome (the closer to NP gene in the gene arrangement on the wild type virus genome), the higher the expression level of the inserted gene will be. To achieve a high expression of a foreign gene, it is preferably inserted into the upstream region of the negative stranded genome such as the upstream of the NP gene (3'flanking sequence on the minus strand) , or between NP and P genes. Conversely, the closer the insertion position to the 5'-terminus of the negative strand RNA (the closer to L gene in the gene arrangement on the wild type virus genome), the lower the expression level of the inserted gene will be. To reduce the expression of a foreign gene, it should be inserted as close as possible to the 5' terminus on the negative strand, that is, downstream of the L gene in the wild type virus genome (5' flanking region of the L gene on the negative strand) or upstream of the L gene (3' flanking region of L gene on the negative strand) . Thus, the insertion position of a foreign gene can be properly adjusted so as to obtain a desired expression level of the gene or optimize the combination of the insert with the surrounding virus genes. To enable simple insertion of a foreign gene, a cloning site may be designed at the position of insertion. For example, the cloning site may be a recognition sequence of restriction enzymes. The restriction sites in the vector DNA encoding genome can be used to insert a foreign gene. The cloning site may be a multicloning site that contains recognition sequences for multiple restriction enzymes. The vector of the present invention may have other foreign genes at positions other than that used for the above insertion.

Construction of recombinant Sendai virus vector having a foreign gene can be performed, for example, as follows according to the method described in Kato, A. et al., EMBO J. 16: 578-587 (1997) ; and Yu, D. et al., Genes Cells 2: 457-466 (1997).

First, a DNA sample containing a cDNA sequence encoding a desired foreign gene is prepared. It is preferable that the concentration of the sample is 25 ng/µl or higher and that it can be detected as a single plasmid by electrophoresis. The following description is an example where a foreign gene is inserted into the *Not*I site of virus genomic DNA. If the desired cDNA sequence contains a *Not*I site, the site is desirably removed in advance by altering the nucleotide sequence using known methods, such as site-directed mutagenesis, while maintaining the encoded amino acid sequence. The desired DNA fragment is amplified by PCR from the DNA sample. In order to obtain fragments having *Not*I sites at both ends and to add a single copy of the transcription end sequence (E) , intervening sequence (I), and transcription start sequence (S) of the Sendai virus (EIS sequence) to one end, a primer pair, i.e., synthesized DNA sequences as a forward primer and a reverse primer (antisense strand), which comprises a *Not*I recognition site; E, I, and S sequences; and part of the desired gene, is prepared.

For example, the forward synthetic DNA sequence contains two or more nucleotides at the 5'-terminus to ensure digestion with *Not*I (preferably 4 nucleotides not containing a sequence derived from the *Not*I recognition site, such as, GCG and GCC; more preferably ACTT). To the 3'-terminus of the sequence, the *Not*I recognition sequence GCGGCCGC is added. Furthermore, to the 3'-terminus any 9 nucleotides or 9 plus multiples of 6 nucleotides are added as a spacer. Furthermore, a sequence of approximately 25 nucleotides corresponding to the ORF of the desired cDNA starting with the initiation codon ATG is added to the 3'-terminus. The 3'-terminus of the forward synthetic oligo DNA containing approximately 25 nucleotides of the desired cDNA is preferably selected so that the last nucleotide is G or C.

The reverse synthetic DNA sequence contains two or more nucleotides at the 5'-terminus (preferably 4 nucleotides not containing a sequence derived from the *Not*I recognition site, such as, GCG and GCC; more preferably ACTT). The NotI recognition sequence GCGGCCGC is added to the 3'-terminus of the sequence. Furthermore, a spacer oligo DNA is added to the 3'-terminus in order to adjust the length of the primer. The length of the oligo DNA is designed so that it is a multiple of 6 nucleotides including the *Not*I recognition sequence GCGGCCGC, the sequence complementary to the cDNA, and the EIS sequence derived from the Sendai virus genome as described below (so-called "rule of six"; Kolakofski, D. et al., J. Virol. 72, 891-899 (1998)). Furthermore, to the 3'-terminus of the added sequence, complementary sequence to the S sequence of the Sendai virus (preferably 5'-CTTTCACCCT-3'), the I sequence (preferably 5'-AAG-3'), and complementary sequence to the E sequence (preferably 5'-TTTTTCTTACTACGG-3') are added. Finally, a sequence of the complementary strand of the desired cDNA, which sequence is selected so that the last nucleotide becomes G or C, is added as the 3'-teminus of the reverse synthetic oligo DNA, wherein the last nucleotide is approximately 25 nucleotides upstream from the termination codon of the cDNA.

PCR can be performed by common methods in the art, such as, ExTaq polymerase (TaKaRa). Vent polymerase (NEB) may be preferably used and the amplified fragment digested with *Not*I and inserted into the *Not*I site of the plasmid vector pBluescript. The nucleotide sequence of the obtained PCR product is checked with an automated DNA sequencer and a plasmid having the correct sequence is selected. The insert is excised from the plasmid by *Not*I digestion, and subcloned into the *Not*I site of the plasmid comprising genomic cDNA. Alternatively, the PCR products may be directly cloned into the *Not*I site without using pBluescript vector to obtain recombinant SeV cDNA.

A viral genome-encoding DNA is ligated with an appropriate transcriptional promoter to construct a vector DNA. The resulting vector is transcribed *in vitro* or in cells and RNP is reconstituted in the presence of viral L, P, and NP proteins to produce a viral vector comprising the RNP. Reconstitution of a virus from viral vector DNA can be performed according to known methods (WO97/16539; WO97/16538; Durbin, A. P. et al. , Virol. 235, 323-332 (1997); Whelan, S. P. et al., Proc. Natl. Acad. Sci. USA 92, 8388-8392 (1995); Schnell, M. J. et al., EMBO J. 13, 4195-4203 (1994); Radecke, F. et al., EMBO J. 14, 5773-5784 (1995); Lawson, N. D. et al., Proc. Natl. Acad. Sci. USA 92, 4477-4481 (1995); Garcin, D. et al., EMBO J. 14, 6087-6094 (1995); Kato, A. et al., Genes Cells 1, 569-579 (1996); Baron, M. D., and Barrett, T., J. Virol. 71, 1265-1271 (1997); Bridgen, A., and Elliott, R. M., Proc. Natl. Acad. Sci. USA 93, 15400-15404 (1996)). These methods enable reconstitution of Paramyxovirus vectors including the parainfluenza virus, vesicular stomatitis virus, rabies virus, measles virus, rinderpest virus, and Sendai virus vectors from DNA. When the F, HN, and/or M genes are deleted from the virus vector DNA, infective virus particles will not be formed. However, it is possible to generate infective virus particles by introducing these deleted genes and/or genes encoding an envelope protein from another virus into the host cells and expressing them.

Methods for introducing vector DNA into desired cells include: (1) a method involving the step of forming DNA precipitates that can be incorporated into desired cells; (2) a method involving the step of preparing a complex that comprises positively charged DNA having low cytotoxicity and which is suitable for the incorporation into desired cells; and (3) a method using electrical pulse for instantaneously opening a pore in the desired plasma membrane large enough for a DNA molecule to pass through.

A variety of transfection reagents can be used in (2), for instance, DOTMA (Boehringer), Superfect (QIAGEN #301305), DOTAP, DOPE, and DOSPER (Boehringer #1811169). For (1), transfection using calcium phosphate can be used. In this method, DNA incorporated by cells is taken up into phagocytic vesicles, but it is known that a sufficient amount of DNA is also introduced into the nucleus (Graham, F. L., and van Der Eb, J., Virol. 52, 456 (1973); Wigler, M., and Silverstein, S., Cell 11, 223 (1997)). Chen and Okayama (Chen, C., and Okayama, H., Mol. Cell. Biol. 7, 2745 (1987)) studied the optimization of the transfer technology and reported that: (a) maximal efficiency is obtained when cells and precipitates are incubated under 2 to 4% CO₂ at 35°C for 15 to 24 hr; (b) circular DNA has higher activity than linear DNA; and (c) the optimal precipitates are formed when the DNA concentration in the mixed solution is 20 to 30 µg/ml. The method of (2) is suitable for transient transfection. A classic transfection method wherein DEAE-dextran (Sigma #D-9885; M. W. 5 x 10⁵) is mixed with DNA at a desired concentration ratio is known in the art. Because most complexes are degraded in the endosome, chloroquine may be added to enhance the transfection efficiency (Calos, M. P., Proc. Natl. Acad. Sci. USA 80, 3015 (1983)). The method of (3), called electroporation, may be more broadly applied than the method (1) or (2) because it can be applied to any kind of cell. High transfection efficiency can be obtained by optimizing the duration of pulse currents, the form of pulse, the strength of the electrical field (gap between electrodes and voltage), conductivity of buffer, DNA concentration and cell density.

Among the above-mentioned three methods, the method using transfection reagent (i.e., method (2)) is suitable for the present invention due to its ease of performance, whereby testing of a large number of samples using a large amount of cells is possible. Preferred transfection reagents include, Superfect Transfection Reagent (QIAGEN, #301305) and DOSPER Liposomal Transfection Reagent (Boehringer Mannheim #1811169).

Reconstitution from cDNA can be performed as follows:
LLC-MK2, a cell line derived from monkey kidney, is cultured in a 24-well to 6-well plastic plate or in a 100-mm Petri dish in minimum essential medium (MEM) containing 10% fetal calf serum (FCS) and antibiotics (100 units/ml penicillin G and 100 µg/ml streptomycin) to be 70 to 80% confluent. Cells are then infected, for instance, at 2 PFU/cell with recombinant vaccinia virus vTF7-3 expressing T7 polymerase, which virus has been inactivated by a 20-minute UV exposure in the presence of 1 µg/ml psoralen (Fuerst, T. R. et al., Proc. Natl. Acad. Sci. USA 83, 8122-8126 (1986); and Kato, A. et al., Genes Cells 1, 569-579 (1996)). The amount of psoralen and the duration of UV exposure can be optimized. One hour after infection, cells are transfected by, for example, lipofection using Superfect (QIAGEN) with 2 to 60 µg, or more preferably 3 to 5 µg of the above recombinant Sendai virus cDNA together with expression plasmids for virus proteins (24-0.5 µg pGEM-N, 12-0.25 µg pGEM-P, and 24-0.5 µg pGEM-L, or more preferably 1 µg pGEM-N, 0.5 µg pGEM-P, and 1 µg pGEM-L) (Kato, A. et al., Genes Cells1, 569-579 (1996)) that function in trans and are required for producing a full length Sendai virus genome. The transfected cells are cultured in serum free MEM containing, if desired, 100 µg/ml rifampicin (Sigma) and cytosine arabinoside (AraC) (Sigma), preferably 40 µg/ml AraC, so that the drug concentration is adjusted to be optimal to minimize the cytotoxicity of the vaccinia virus and maximize the recovery of the virus (Kato, A. et al., Genes Cells 1, 569-579 (1996)). Cells are cultured for 48 to 72 hr after transfection, then collected and lysed through three cycles of freeze-thawing. The cell lysates are transfected into LLC-MK2 cells, and after a 3- to 7-day culture the culture medium is collected. To reconstitute a virus vector lacking a gene encoding an envelope protein that is incapable of replication, the vector may be transfected into LLC-MK2 cells expressing the envelope protein, or co-transfected with an expression plasmid for the envelope protein. Alternatively, transfected cells can be overlaid and cultured on LLC-MK2 cells expressing the envelope protein to propagate a deletion virus vector (see International Application Numbers PCT/JP00/03194 and PCT/JP00/03195). The virus titer of the culture supernatant can be determined by measuring hemagglutinin activity (HA). The HA may be determined by the "endo-point dilution method" (Kato, A. et al., Genes Cells 1, 569-579 (1996)). The obtained virus stock can be stored at -80°C.

According to the present invention, host cells are not limited to any special type of cells as long as the virus vector can be reconstituted in the cells. Host cells may include LLC-MK2 cells; CV-1 cells derived from monkey kidney; cultured cell lines, such as, BHK cells derived from hamster kidney; and human-derived cells. Furthermore, to obtain a large quantity of Sendai virus vector, embryonated chicken eggs may be infected with virus vectors obtained from the above host cells and the vectors can be amplified. The method of producing virus vectors using chicken eggs is well established (Advanced protocols in neuroscience study III, Molecular physiology in neuroscience., Ed. by Nakanishi et al., Kouseisha, Osaka, 153-172 (1993)). For example, more specifically, fertilized eggs are incubated for 9 to 12 days at 37 to 38°C in an incubator to grow the embryos. Virus vectors are inoculated into the allantoic cavity, and eggs are further incubated for several days to propagate the vectors. Conditions like duration of incubation may vary and depend upon the type of recombinant Sendai virus used. Then, allantoic fluids containing viruses are recovered. The Sendai virus vector is separated and purified from the allantoic fluid sample according via a standard method (Tashiro, M. , Protocols in virus experiments., Ed. by Nagai and Ishihama, MEDICAL VIEW, 68-73 (1995)).

To prepare a deletion virus vector, for example, two different virus vectors with genomes lacking different envelope gene, respectively, are transfected into a cell. As a result, envelope proteins encoded by the deleted genes are supplied through the expression from one of the vectors, and this mutual complementation permits the generation of infective virus particles that can replicate and propagate. Thus, two or more of the virus vectors of the present invention may be simultaneously inoculated in combination that complements each other and produces a mixture of each envelope deletion virus vector at a low cost and on a large scale. Because these viruses lacking an envelope gene have a smaller genome, they allow insertion of a longer foreign gene compared to those without deletion. In addition, the co-infecting ability of these intrinsically non-infective viruses can be hardly retained after dilution outside the cell; which means that such virusesare sterilized outside the cell and are less harmful to the environment.

Gene therapy can be conducted by preparing a viral vector using, as a foreign gene, a gene suitable for the treatment of a disease and then administering the vector. The viral vector of the present invention can be used in gene therapy to express a foreign gene for which a therapeutic effect is expected or an endogenous gene whose *in vivo* expression is impaired in a patient. Expression of the gene may follow either direct administration or indirect (*ex vivo*) administration of the viral vector. There are no limitations on any specific type of foreign gene, as it may include not only nucleic acids encoding proteins but also nucleic acids which do not encode any proteins (e.g., antisense or ribozyme).

The Paramyxovirus vector of the present invention can be formulated as a composition together with a desired pharmaceutically acceptable carrier. Herein, the phrase "pharmaceutically acceptable carrier" is defined as materials that can be administered with a vector and does not inhibit gene transfer achieved by the vector. For instance, the Paramyxovirus vector of the invention may be appropriately diluted with saline, phosphate buffered saline (PBS), etc. to make a composition. If the Paramyxovirus vector of the invention is propagated in chicken eggs, the composition may contain allantoic fluids. Also, the composition may contain carriers or media such as deionized water or 5% dextrose aqueous solution. It may further contain stabilizers, and antibiotics, etc.

A foreign gene carried by the Paramyxovirus vector that is obtained according to the above-described method can be expressed in skeletal muscle by transferring the Paramyxovirus vector or a composition comprising the vector into the skeletal muscle.

Moreover, prior to the administration of the virus vector, bupivacaine, which is known to enhance the expression of transgenes by inducing muscle regeneration, may be administered.

According to the present invention, there is no limitation on the type of gene to be transferred using Paramyxovirus of the present invention. Such genes may encode naturally occurring proteins or artificial proteins. Naturally occurring proteins include, for example, hormones, cytokines, growth factors, receptors, enzymes, and peptides. Such proteins can be secretory proteins, transmembrane proteins, cytoplasmic proteins, nuclear proteins, etc. Artificial proteins include, for example, fusion proteins, such as chimeric toxin; dominant negative proteins (including, soluble molecules of receptors and membrane-bound dominant negative receptors); deficient-type cell-adhesion molecules; and cell-surface molecules. Furthermore, secretory signal, membrane localization signal, or nuclear translocation signal may be added to such proteins. A gene to be introduced may be a gene which is not originally expressed in skeletal muscle. Alternatively, a gene which is normally expressed in skeletal muscle can be introduced for over-expression. It is also possible to suppress the functions of an undesirable gene expressed in skeletal muscle by introducing an antisense RNA molecule, or a ribozyme that cleaves RNA.

The vector of the present invention can be applied to gene therapy for various diseases. Such gene therapy can be carried out, for example, to compensate for the expression defect in cells due to gene deficiency, to confer a new function by introducing a foreign gene into cells, or to suppress an undesirable activity in cells by introducing a gene that suppresses the activity of a certain gene.

Examples of diseases to be treated by gene therapy include muscular dystrophy; myositis; myopathy; and myopathy and cardiomyopathy following myocardial infarction. Examples of genes effective for the treatment of muscular dystrophy include dystrophin and IGF-I; those for myositis, IGF-I and FGF; and those for myopathy, IL-10, IL-12, and IL-6.

A preferable example of a gene that is introduced by the Paramyxovirus of the present invention includes a gene encoding an insulin-like growth factor. As a result, a significant increase in regenerating myofibers and splitting myofibers (they indicate hypertrophy) , as well as an increase in the total number of myofibers can be expected. Thus, Paramyxovirus into which a gene that encodes an insulin-like growth factor has been introduced is expected to be applicable for the treatment of neuromuscular disorders. Examples of neuromuscular disorders include nerve fiber breakage caused by injury, amyotrophic lateral sclerosis, and spinal amyotrophy.

Gene therapy can be carried out by administering a composition containing the Paramyxovirus vector *in vivo*, either intramuscularly or extramuscularly, and expressing the foreign gene in skeletal muscle. In addition, it may also be administered *ex vivo*. Methods for introducing the vector into skeletal muscle include methods wherein the vector is introduced transcutaneously and methods wherein the vector is directly introduced by incision of the skin. When introducing the vector, one should be careful not to damage the epimysium.

The Paramyxoviral vector is administered into skeletal muscle at a sufficient dose that ensures the introduction of an effective amount of the vector into skeletal muscle. The term "effective amount," as employed herein refers to an amount that ensures the introduction of the gene into skeletal muscle by the method of the present invention to produce a desired therapeutic or preventive effect (at least in part) . The administration of an effective amount of the Paramyxoviral vector of the present invention comprising a desired gene induces alterations in the phenotypes of the cells where the vector has been introduced and/or the surrounding skeletal muscle. Preferably, the administration of an effective amount of the vector of the present invention comprising the desired gene to skeletal muscle leads to the introduction of the gene into a significant number of cells in the skeletal muscle as well as to the induction of alterations in the phenotypes of the cells. The phrase "a significant number of cells" means that the gene has been introduced via the vector of the present invention into at least about 0.1%, preferably about 1% or more, more preferably about 5% or more, still more preferably about 10% or more, most preferably about 20% or more of target skeletal muscle at the administration site.

The achievement of gene transfer into cells can be confirmed via assay methods known to those skilled in the art. For example, the transcript of a gene can be detected, by Northern hybridization, reverse transcriptase-polymerase chain reaction (RT-PCR), or RNA protection assay. The detection by Northern hybridization, RT-PCR, etc. can also be carried out *in situ*. The detection of the translation product can be carried out using antibody by Western blotting, immunoprecipitation, RIA, enzyme-linked immunosorbent assay (ELISA), pull-down assay, etc. To easily detect the achievement of gene transfer, the protein to be expressed can be tagged or a reporter gene can be inserted so as to ensure its expression. The reporter gene includes, but is not limited to, genes encoding β-galactosidase, chloramphenicol acetyltransferase (CAT), alkaline phosphatase, and green fluorescent protein (GFP).

The dose of the vector and the route of administration can be appropriately determined by those skilled in the art, and may vary depending on the disease, body weight, age, sex, and symptom(s) of the patient, the purpose of administration, the kind of transgene, etc. The concentration of the vector (with pharmaceutically acceptable carriers) may be preferably within the range of approximately 10⁵ PFU/ml to 10¹¹ PFU/ml, more preferably approximately 10⁷ PFU/ml to 10⁹ PFU/ml, and most preferably approximately 1 x 10⁸ PFU/ml to 1 x 10⁹ PFU/ml.

The composition of the present invention comprising the virus vector may be administered into subjects, including all mammalian animals, such as humans, monkeys, mice, rats, rabbits, sheep, cattle, and dogs.

### Brief Description of the Drawings

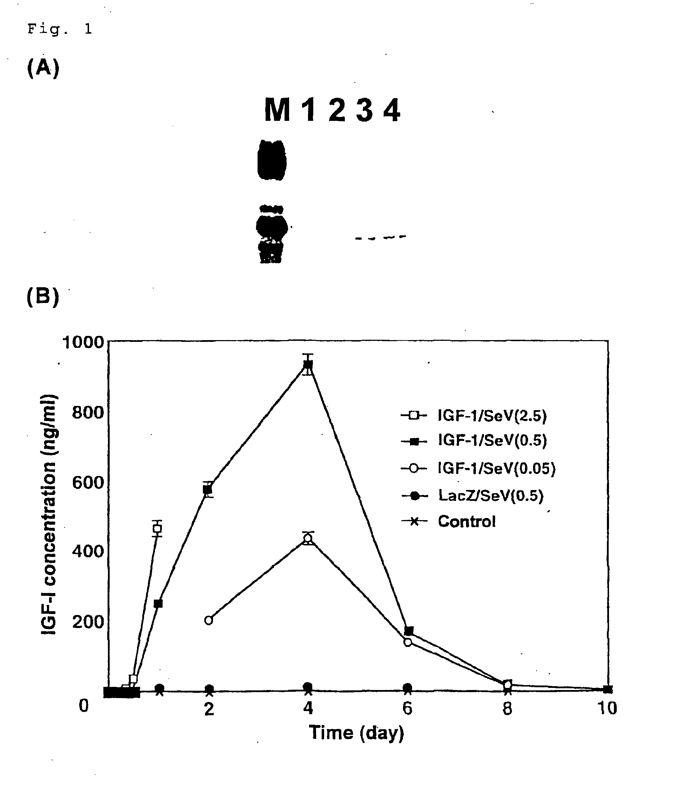

Fig. 1 depicts a photograph and graph showing *in vitro* expression of hIGF-I. (A) The photograph showing the expression of hIGF-I in the culture supernatant of virus infected L6 cells. Samples from culture supernatant with following conditions were analyzed by Western blotting (1= virus free, 2= LacZ/SeV (moi=0.1, 3 days) 3= IGF-I/SeV (moi=0.1, 3 days), 4= IGF-I/SeV (moi=0.1, 4 days). (B) The graph showing time- and moi-dependent expression of hIGF-I derived from hIGF-I/SeV infected L6 cells. Supernatants from cells infected with different moi were taken at different time points, and assayed for hIGF-I quantities using ELISA kit.

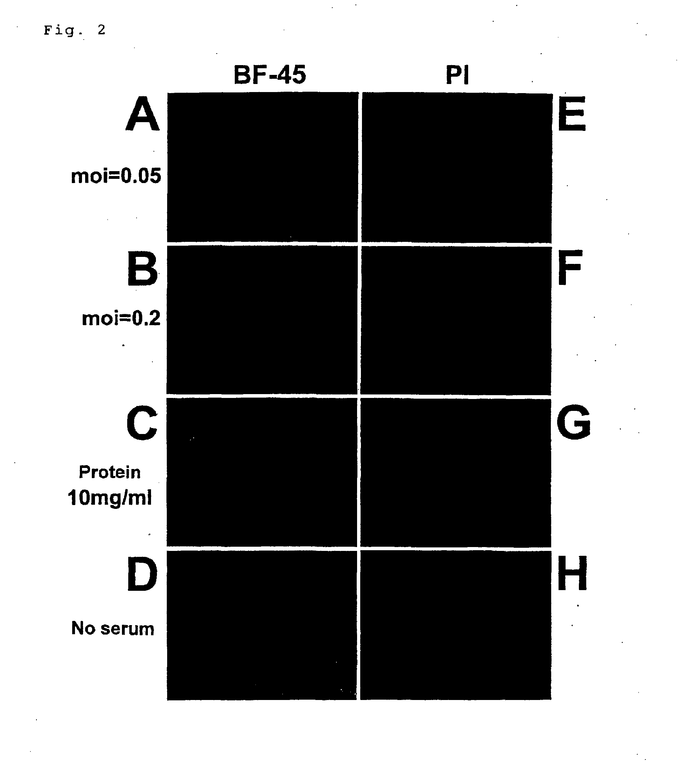

Fig. 2 depicts photographs showing myogenic differentiation and hypertrophy of L6 cells enhanced by SeV-mediated hIGF-I.
   Control cells cultured in serum-free media (differentiation media) for 4 days. The virus infected cells were infected at either moi=0.05 or 0.2, and cultured in serum-free media for 4 days. After fixation, the myotubes were treated with monoclonal antibody against the myosin heavy chain embryonic subunit (MAb BF-45) and other cells were subjected to nuclear staining. The viral infected cells shows moderate myotube hypertrophy at moi=0.05 and much more pronounced myotube hypertrophy at moi=0.2 than those cultured under serum free condition alone. Nuclear organization was observed in the viral infected cells, which was grouped in the middle of the myofibers.

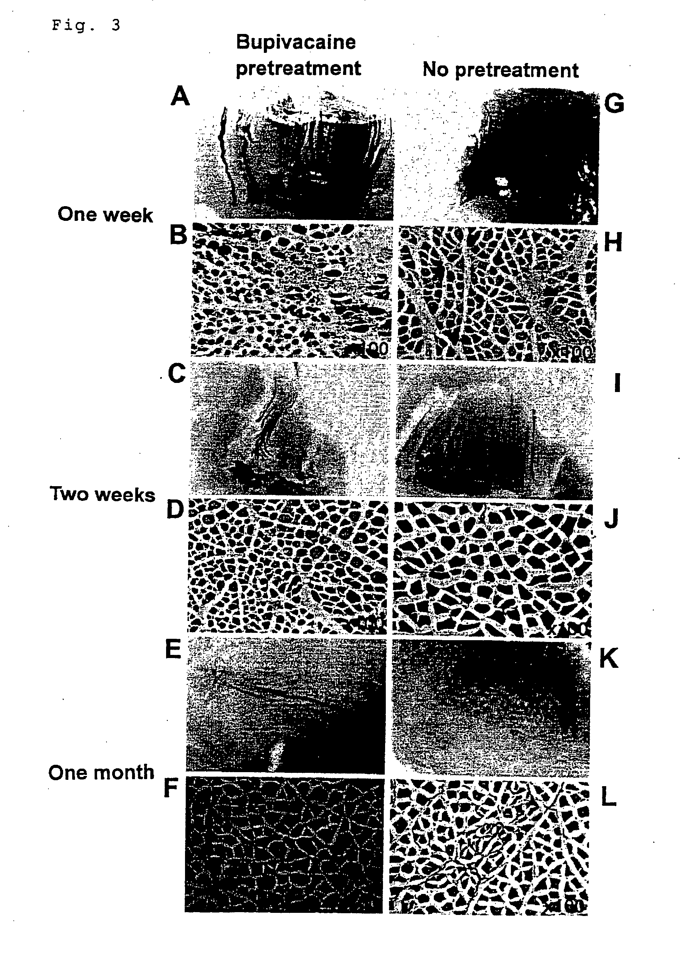

Fig. 3 depicts photographs showing transducibility of SeV to skeletal muscle *in vivo.*
   With/without pre-treatment with myonecrotic agent (bupivacaine), 200 µl of recombinant SeV (5 x 10e7 PFU) carrying the LacZ reporter gene (LacZ/SeV) was injected in the tibialis anterior muscle of mature rat. After indicated days as follows, the muscle was excised and stained for the β-galactosidase activity; A and B: bupivacaine (7 days), C and D: bupivacaine (14 days), E and F: bupivacaine (30 days), G and H: no bupivacaine (7 days), I and J: no bupivacaine (14 days), and K and L :no bupivacaine (30 days).

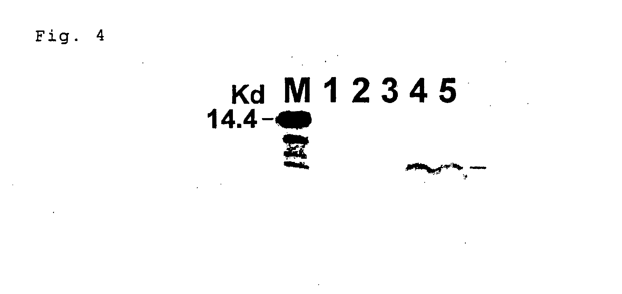

Fig. 4 depicts a photograph showing the expression of hIGF-I in the tibialis anterior muscle 7 days after virus injection analyzed by Western blotting with anti-hIGF-I antibody. 200 µl each of either LacZ/SeV (2 x 10e8 PFU) or hIGF-I/SeV (2 x 10e8 PFU) was injected in the tibialis anterior muscle. 7 Days later, 300 µl of tissue extraction solution was obtained from 100 µg of excised frozen muscle tissue. Proteins in 50 µl of the extraction solution(corresponding to 16.7 µg tissue) were precipitated with cold acetone, and then were subjected to Western blot analysis with anti-hIGF-I antibody. (M: marker proteins, 1: no-treatment, 2: LacZ/SeV (#1 animal) , 3: LacZ/SeV (#2 animal), 4: hIGF-I/SeV (#3 animal), 5: hIGF-I/SeV (#4 animal)).

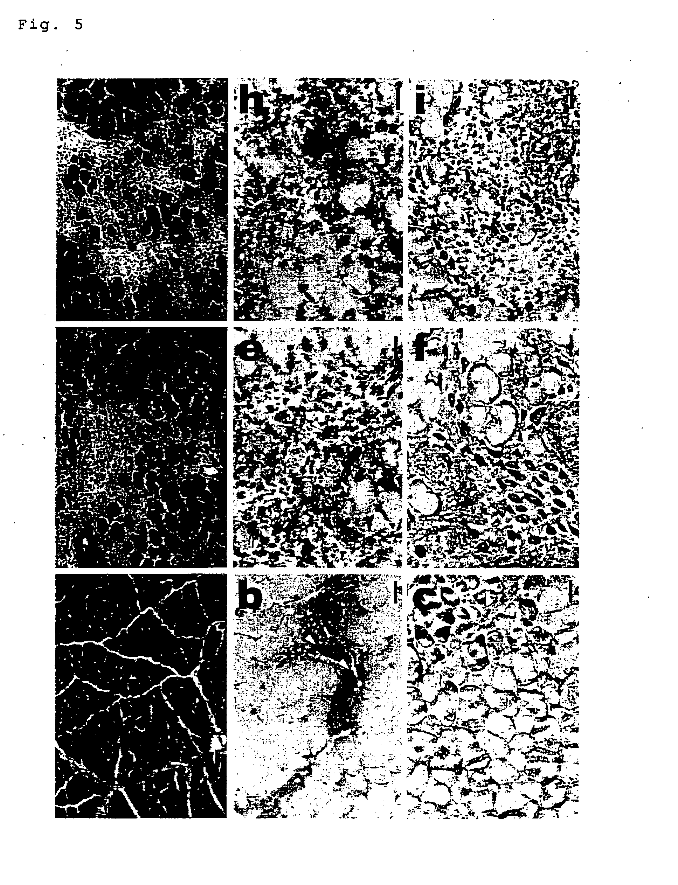

Fig. 5 depicts photographs showing the expression of hIGF-I in the tibialis anterior muscle 7 days after virus injection. Regeneration of the tibialis anterior muscle. 200 µl each of LacZ/SeV (2 x 10e8 PFU) and hIGF-I/SeV (2 x 10e8 PFU) was injected in the right-or left-tibialis anterior muscles, respectively. The excised muscle transverse sections were treated with hematoxylin-eosin (HE) (a, d, and g), with acid phosphatase to detect macrophage (b, e, and h), and with BF-45 (c, f, and i). Macrophage and BF-45 positive cells are shown with white and black arrowheads, respectively.

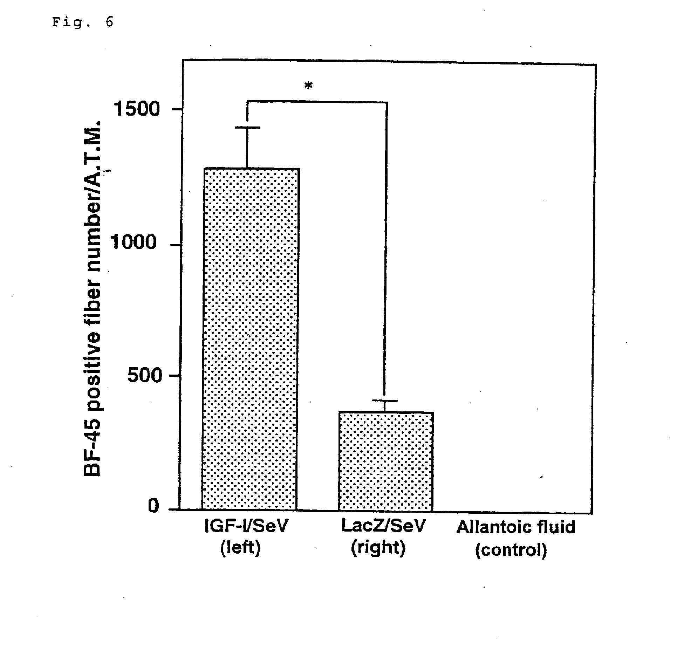

Fig. 6 depicts a graph showing the expression of hIGF-I in the tibialis anterior muscle 7 days after virus injection. The number of myofiber expressing embryonic MyHC. The total number of positive embryonic MyHC cells was counted (n=4) , and the results are expressed as the mean±SD (n=4). The single asterisk denotes P<0.01 for paired comparisons (Student's t test).

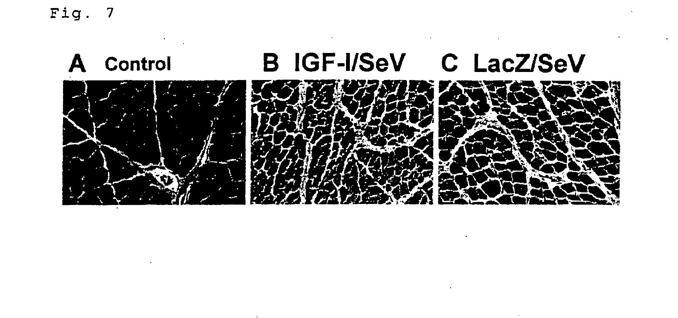

Fig. 7 depicts photographs showing the regeneration of the tibialis anterior muscle 14 days after virus injection. 200 µl each of LacZ/SeV (2 x 10e8 PFU) and hIGF-I/SeV (2 x 10e8 PFU) was injected in the right- or left-tibialis anterior muscles, respectively. The transverse sections of excised muscle were treated with hematoxylin-eosin. (A: allantoic fluid only, B: IGF-I/SeV (left), C: LacZ/SeV (right)).

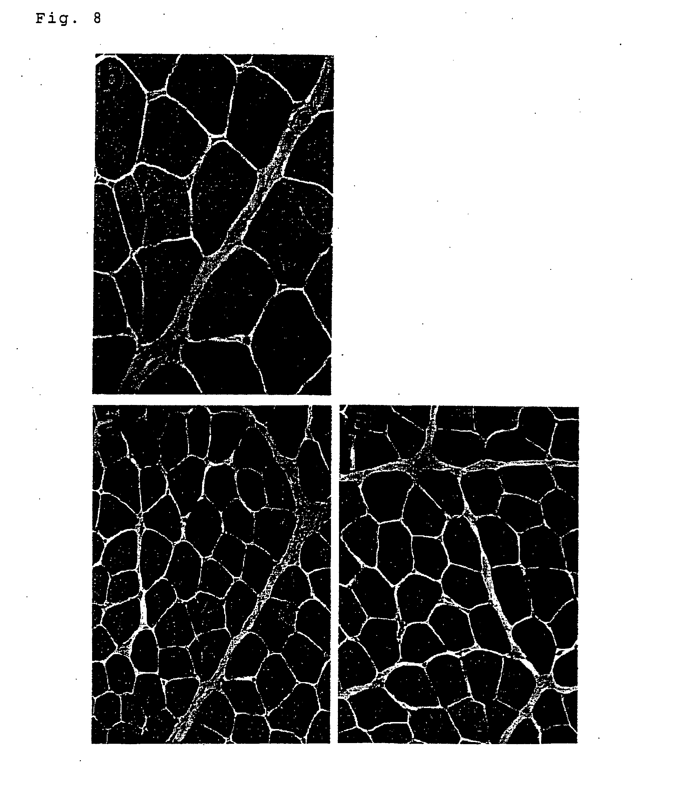

Fig. 8 depicts photographs showing the regeneration of the tibialis anterior muscle 30 days after virus injection. Almost no inflammatory reaction was seen in the section. IGF-I/SeV treated muscle showed splitting phenomena due to hypertrophy (Fig. 8a and b). Splitt positions are shown with arrowheads. The regeneration myofibers treated with LacZ/SeV returned nearly to normal (Fig. 8c).

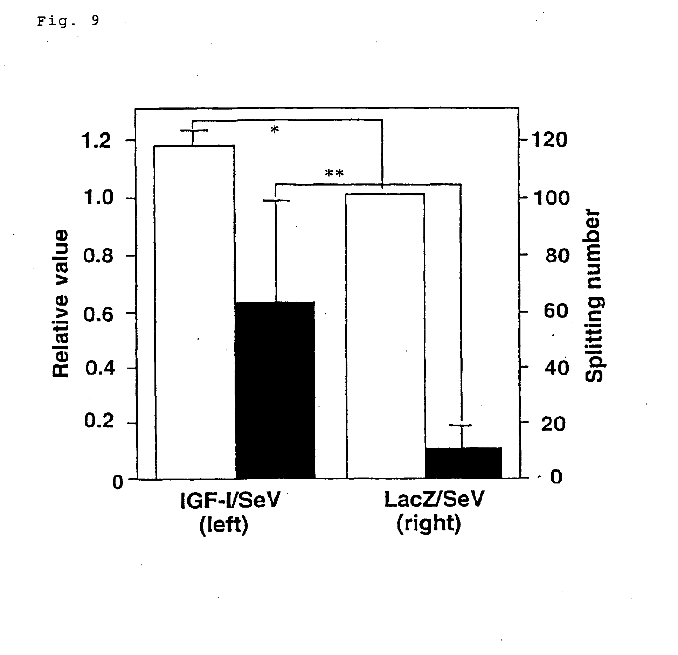

Fig. 9 depicts a graph showing the regeneration of the tibialis anterior muscle 30 days after virus injection. Effect of hIGF-I expression on the number of myofibers (open bars) and number of splitting fibers (closed bars). The total number of fibers of the LacZ/SeV-treated tibialis anterior muscle of each animal in group 1 served as the control (the value defined as 1) for hIGF/SeV-treatment (the results expressed as relative values to the control) (open bars) . The average relative value (n=4) is plotted (±SD). Single asterisk denotes the P<0.03 for paired comparisons (Student's t test). The number of splitting fibers was counted, and the results are expressed as the mean±SD (n=4). Double asterisks denote the P<0.01 for paired comparisons (Student's t test).

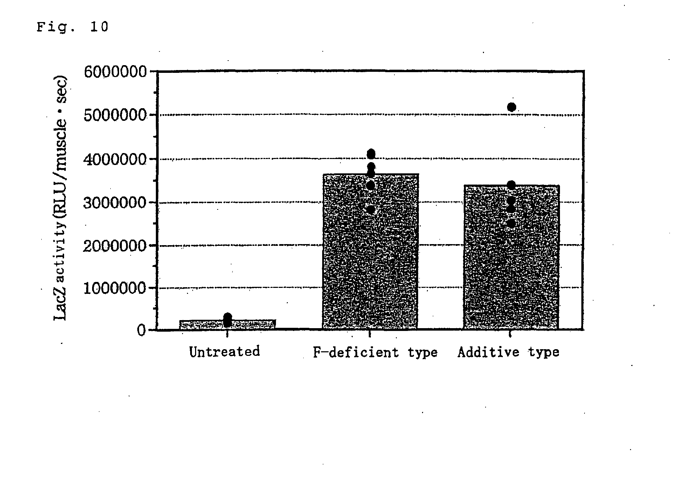

Fig. 10 depicts a graph showing the comparison of gene introduction in mouse leg muscle (after 3 days). The titer was 4 x 10⁷ CIU/head (dose: 100 µl, administration: twice).

### Best Mode for Carrying out the Invention

The present invention will be explained in detail below with reference to Examples, but it is not to be construed as being limited thereto.

### [Example 1] Construction of Sendai virus vector

The human IGF-I open reading frame was amplified by PCR from a human cDNA library (Gibco BRL, Rockville, MD) with primers, 5'-ATCCGAATTCGCAATGGGAAAAATCAGCAGTC-3' (SEQ ID NO: 1) and 5'-ATCCGAATTCCTACATCCTGTAGTTCTTGTTTCCTGC-3' (SEQ ID NO: 2), based on the DNA sequence of Accession number X00173 (Nature 306, 609-611 (1983)). The resulting PCR product was cloned at the *Eco*RI site of pBluescript II, and then was sequenced. The product with the correct sequence of hIGF-I gene was re-amplified with primers, containing SeV-specific transcriptional regulation signal sequences, 5'-ATCCGCGGCCGCCAAAGTTCAGCAATGGGAAAAATCAGCAGTCTTC-3' (SEQ ID NO: 3) and 5'-ATCCGCGGCCGCGATGAACTTTCACCCTAAGTTTTTCTTACTACGGCTA CATCCTGTAGTTCTTGTTTCCTGC-3' (SEQ ID NO: 4), and was cloned to generate pIGF-I/SeV in the *Not*I site of the parental pSeV18+b(+), which was constructed to prepare exact SeV full-length antigenomic plus sense RNA of 15402 nucleotide. The resulting pIGF-I/SeV was transfected into LLCMK2 cells infected with vaccinia virus vTF7-3, expressing T7 polymerase. T7-driven full-length recombinant IGF-I/SeV RNA genome was encapsulated with N, P, and L proteins, which were dried from respective cotransfected plasmids. After a 40-hr incubation, the transfected cells were injected into embryonated chicken eggs to amplify the recovered virus.

### [Example 2] In vitro hIGF-I expression

IGF-I has been shown to be closely related to proliferation, differentiation, and hypertrophy in both rats (J. Biol. Chem. 272, 6653-6662 (1997); J. Cell. Biol. 135, 431-440 (1996)) and mice (J. Biol. Chem. 264, 13810-13817 (1989)) cell lines. The present inventors investigated whether recombinant human IGF-I can induce morphological changes of L6 cells (neonatal rat myoblast cell line) via SeV-mediated gene transfer. The newly constructed recombinant SeV harboring human IGF-I (hIGF-I) gene, designated as hIGF-I/SeV, and SeV encoding β-galactosidase (LacZ/SeV) were used for investigating the following facts : (1) IGF-I expression in supernatant (Western blotting analysis), (2) kinetics of IGF-I expression in supernatant (ELISA assay) , and (3) morphological change of L6 cells.

### In vitro study

Differentiation into myotube cells of L6 cells was suppressed by culturing them in DMEM medium with 20% FCS and penicillin/streptomycin. The cells were infected with virus in serum-free DMEM or without serum followed by culturing under differentiating conditions. For immunoblotting, 100 µl of supernatant were concentrated to 10 µl with 2 volumes of cold-acetone, were separated by sodium dodecyl sulfate-polyacrylamide gel electrophoresis (SDS-PAGE) using a 15-25% resolving gel, and then were transferred to a polyvinylidene-difluoride membrane (DAIICHI PURE CHEMICALS, Tokyo) . Primary antibody reaction was performed with anti-human IGF-I monoclonal antibody (Diagnostic Systems Laboratories, Webster, TX), and horseradish peroxidase-labeled antibody was used for the second reaction. Complexes were visualized using enhanced chemiluminescence (ECL, Amersham, UK). ELISA assay was conducted to measure the IGF-I level in the culture supernatant. The assay was basically performed following the manufacturer's recommendations (R&D systems, Minneapolis, MN).

As a result, (1) reaction bands with a molecular mass of 12-13 kDa were observed for the culture supernatants of L6 cells infected with hIGF-I/SeV under the conditions of a multiplicity of 0.1 for 3 and 4 days (Fig. 1A, lanes 3, and 4) . This protein specie was absent in LacZ/SeV infected supernatant. The result demonstrates that hIGF-I was produced from the recombinant SeV and was secreted into the culture supernatant.

(2) Fig. 1B shows the amount of IGF-I expressed in the L6 cell supernatant after viral infection. Under an infectious condition with a multiplicity of 0.05, 48 hr after infection the IGF-I concentration in the supernatant increased to 203 ng/ml without showing cytopathogenic effect (CPE) to the cells, and after 96 hr increased to 435 ng/ml. At a multiplicity of 0.5, 566 ng/ml and 935 ng/ml of IGF-I were secreted into the medium after 48 hr and 96 hr, respectively. Under an infectious condition with a multiplicity of 2.5, IGF-I concentration of 39 ng/ml and 463 ng/ml were detected after 12 hr and 24 hr, respectively. No IGF-I could be observed in the LacZ/SeV infected supernatant. These results show that the gene products were secreted into the culture supernatant via the SeV-mediated gene transfer.

(3) L6 cell line is known to express the IGF-I receptor instead of IGF-I, and therefore is responsive to exogenous IGF-I, which induces hypertrophy (J. Cell. Biol. 135, 431-448 (1996)). The ability of recombinant IGF-I to induce differentiation of L6 was examined. L6 cells were grown in medium containing 20% FCS, and virus-mediated gene transfer was conducted at 80% confluency, and the medium was substituted with serum-free medium or serum-free medium with a definite concentration of hIGF-I protein. 500 µg/ml of bovine serum albumin was added to all samples to suppress adsorption of hormone to the cell surface. After four days, clear differences in cell morphology dependent on the virus infection multiplicity could be observed (Fig. 2). Generated myotubes were treated with myosin heavy chain (MyHC) antibody against the embryonic subunit (MAbBF45). The bound antibody was visualized with Alexa Flour™ 568 goat anti-mouse IgG (H+L) conjugate. Nuclei of the cells were visualized by propidium iodine staining. The cells cultured in the media containing hIGF-I at the concentrations of a multiplicity of 0.05 (Fig. 2A and B) and 0.2 (Fig. 2B and F) exhibited larger myotubes, multiple nuclear in the middle of the myofibers, and hypertrophy (i.e., increase in size and width of the myotube) (Fig. 2A, B, E, and F), compared to cells cultured in both serum and hIGF-I free condition (Fig. 2D and H). Moreover, the morphological changes induced by the viral infection was consistent with that induced by the IGF-I protein (Fig. 2C and G). These results indicate that the efficacy of the recombinant hIGF-I from SeV was the same as that of the IGF-I protein, and that the recombinant hIGF-I also shows biological function *in vitro* and induced myogenesis of the neonatal rat myoblast L6 cells.

### [Example 3] In vivo LacZ reporter gene expression

Sprague-Dawley rats (male, 6 week old, 160-180 g) were anesthetized by intraperitoneal injection of pentobarbital sodium (50 mg/kg). The hind limbs were shaved and scrubbed with ethanol, and 1.5-cm skin incisions were made to expose the tibialis anterior muscles. For LacZ reporter gene transfer study, 200 µl of LacZ /SeV (5 x 10e7 PFU) were injected into the mid belly of the left tibialis anterior muscle. Transgene expression was observed by X-gal staining. For bupivacaine treated animals, 3 days prior to vector injection, 200 µl of 0.5% bupivacaine solution was injected into the tibialis anterior muscle .

As a result, one week after LacZ/SeV injection, the muscle tissue pre-treated with bupivacaine showed a high level X-gal staining (Fig. 3A and B) compared with the untreated muscle (Fig. 3G and H). Some X-gal positive fibers were observed among comparatively small myofibers around necrotic fibers, and infiltrating cells of neutrophils and macrophages in bupivacaine pretreated muscle (Fig. 3B). The small myofibers are considered to be regenerated immature fibers, which were induced by the intramuscular injection of bupivacaine and/or by viral injection in the tibialis anterior muscle of adult rats. Vitadello *et al*. (Hum. Gene Ther. 5, 11-18 (1994)) demonstrated that, 3 days after bupivacaine treatment, muscle show mononucleated cells and small myotubes which can be stained with anti MyHC monoclonal antibody (BF-45), and that the enzymatic activity expressed by the transfection of naked DNA is higher after 3 days than 1 or 7 days after the injury. The small and positive fibers to which SeV could introduce the LacZ gene were considered to be mitotically active myoblasts or immature myotubes emerging during skeletal muscle maturation. In non bupivacaine-treated animals, the necrotic myofiber area induced by viral infection was much smaller than that induced in bupivacaine and virus treated animals. Although the number of positive fibers were less than that in the bupivacaine treated ones, X-gal labeled myofibers could be also obseved in the non bupivacaine-treated myofibers (Fig. 3H). The cells around the transduced myofibers had no central nucleus and also were larger in size than the regenerated myofibers shown in Fig. 3B, indicating that these cells may be mature myofibers. Taken together, the transduced myofibers may be mature myofibers. Therefore, SeV could introduce the genes encoding proteins into rat mature myofibers. The expression of X-gal could be also observed two weeks after the viral injection. The profiles of positive fibers in bupivacaine-treated and non treated animals (Fig. 3C, D, I, and J) were consistent with that of the samples after 7 days described above, though the number of positive fibers was decreased. 30 days after viral injection, X-gal labeled fibers could be observed only in bupivacaine-treated animals (Fig. 3E and F). On the other hand, X-gal labeled fibers could not be observed in myofibers but in interstitial cells of bupivacaine-untreated animals (Fig. 3K and L). These results demonstrates that SeV is infectious to myoblasts during cell division, and post-mitotic immature as well as mature myotubes.

### [Example 4] In vivo IGF-I gene transfer

Since the high level hIGF-I expression *in vitro* and the introduction of the LacZ gene into the regenerated and mature myofibers by SeV-mediated gene transfer *in vivo* was confirmed, as the next step, the present inventors determined whether hIGF-I introduced into rat skeletal muscle via SeV-mediated gene transfer could promote the growth of rat skeletal muscle, such as increase in the number of myofibers and myotube hypertrophy. First the expression of hIGF-I in the tibialis anterior muscle was determined by Western blot analysis. 200 µl of allantoic fluid, viral solvent containing hIGF-I/SeV with 2 x 10e8 PFU (a four times higher PFU than that in previous experiments), and LacZ/SeV with 2 x 10e8 PFU, respectively, were injected into the tibialis anterior muscle of adult Sprague-Dawley rats.

1 week, 2 weeks, and 30 days after injections, the animals were euthanized by a lethal dose of sodium pentobarbital. The tibialis anterior muscles were excised and then subjected to histological staining and Western blotting. Human IGF-I in the rat muscles was assayed by Western blotting. 100 µg of muscle tissue was frozen in liquid nitrogen, and then milled with a mortar and pestle. 500 µl of chilled 1 M acetic acid was added to each tissue. The mixture was homogenized and then left standing on ice for 2hr. After centrifugation at 3,000 x g for 15 min, the supernatant was collected. Fresh 1 M acetic acid was added to the precipitate for reextraction. Then, the two supernatants were combined, frozen at -70°C, lyophilized, and suspended in 300 µl of 50 mM Tris-HCl buffer (pH 7.8). After concentrating the solution from 50 µl to 10 µl, the solution was subjected to Western blotting.

For immunohistochemical staining of regenerating myofibers, excised tibialis anterior muscle that was immediately frozen in isopentane cooled in liquid nitrogen was used. The obtained sample was cut to 10 µm thick sections, and were placed on lysine coated slides. The immunostaining of regenerating myofibers was conducted by primary antibody reaction treatment of the sections with mouse anti-embryonic myosin heavy chain monoclonal antibody (BF-45); incubating with secondary biotinylated anti-mouse IgG (1:100; Vector, Burlingame, CA) for 1 hr at room temperature; and then incubating with streptoavidin-biotin complex (1:200 dilution; Vector, Burlingame, CA) for 1 hr at room temperature for coloring. The avidin-biotin complex was visualized with 0.05 M Tris-HCl buffer (pH 7.6) containing 0.05% 3, 3'-diaminobenzidine tetrahydrochloride and 0.01% hydrogen peroxide. The sections were then counterstained with eosin. The monoclonal antibody BF-45 was developed by Dr. S. Schiaffino (Hum. Gene Ther. 5, 11-18 (1994)), Padova, Italy, and was obtained from the American Type Culture Collection (Manassas, VA). To assess the regenerating effect of hIGF-I, the number of anti-BF-45 immunopositive fibers were counted 7 days after the injection. In addition, to evaluate the hypertrophic effect of hIGF-I, the number of splitting fibers of muscles were counted after 30 days.

As a result, one week after injection, the samples of the tibialis anterior muscle treated with IGF-I/SeV demonstrated a band of a molecular mass of approximately 8-9 kDa with anti-hIGF-I antibody. This value (Fig. 4A, lanes 4 and 5) is not consistent with that observed in the culture supernatant (Fig. 1A). The precursor of hIGF-I, observed in the *in vitro* experiment seem to convert *in vivo* due to protease cleavage to the mature form of hlGF-I reported to be 7.7 kDa (Bio Science, Cytokinin growth factor, Yodosya, pp.104-105).

Next, the effect of hIGF-I on normal mature muscle was examined. Adult rats (6 week old) received injection of (1) hIGF-I/SeV (2 x 10e8 PFU) into the left tibialis anterior muscle and LacZ/SeV (2 x 10e8 PFU) into the right tibialis anterior muscle (group 1); (2) hIGF-I/SeV (2 x 10e8 PFU) into the left tibialis anterior muscle alone (n=12) (group 2); (3) LacZ/SeV (2 x 10e8 PFU) into the left tibialis anterior muscle alone (n=12) (group 3); or (4) allantoic fluid alone into both tibialis anterior muscles (n=3) (group 4).

Seven days after the viral injection of both IGF/SeV and LacZ/SeV in group 1, fiber resolution due to massive necrosis; edema formation in the perimysium; and infiltration of numerous mononuclear phagocytotic macrophages, lymphocytes, and some neutrophils in the extracellular space were detected in the myofibers at the administration site of the remaining sections (Fig. 5d and g). The remaining normal sized myofibers within the necrotic area were invaded by numerous acid phosphatase-positive macrophages (Fig. 5c and h). However, no damage, just appearance of a very small amount of macrophages, could be observed in the muscle treated with the allantoic fluid alone (Fig. 5a and b). Taken together, SeV introduction causes damage and induces necrosis in the infected muscle, followed by the infiltration of macrophages, lymphocytes, and so on, which remove the necrotic muscle. Phagocytosis, however, is a very important event for muscle regeneration, since the regeneration is inhibited by persisting necrotic tissue. After phagocytosis, muscle precursor cells or satellite cells are activated and proliferation of myoblast is known to start (Jikkenigaku, Yodosya, 444-448 (2000.3.)). Moreover, three days after the bupivacaine treatment, the muscle is reported to be composed of mononucleated cells and small myotubes positive for anti-MyHC monoclonal antibody (BF-45), which cells are indices of regenerating myofibers (Hum. Gene Ther. 5, 11-18 (1994)). Very small round myofibers with central nuclei were observed in infiltrating macrophages. Therefore, immunoreaction of these cells against BF-45 was examined. A significantly larger number of BF-45-positive regenerated myofibers was observed in the left tibialis anterior muscle that received hIGF-I/SeV injection (Fig. 5f) in comparison to the right tibialis anterior muscle which received the LacZ/SeV injection (Fig. 5i). As shown in Fig. 6, the average numbers of BF-45 immunopositive fibers in group 1 animals were 446 (n=4) and 1,722 (n=4) for LacZ/SeV (right) and IGF-I/SeV (left), respectively. No BF-45 immunopositive myofiber was detected in the animals treated with allantoic fluid (group 4, Fig. 5c).

14 days after virus vector administration, the number of macrophages dramatically decreased in both hIGF-I/SeV and LacZ/SeV treated muscles (Fig. 7) (groups 1, 2, and 3). In the LacZ/SeV treated muscle, medium myofibers with the same size having central nuclei increased (Fig. 7C). On the other hand, in the IGF-I/SeV treated muscle, a few necrotic fibers remained with scattered macrophages (data not shown) and various sized myofibers including small sized fibers could be observed (Fig. 7B), which indicate continuous formation of new fibers. No infiltration of fibroblasts and consequent collagenization were observed in either the hIGF-I/SeV or LacZ/SeV treated muscles. The space occupied by interstitial cells, mainly macrophages, 7 days after viral induction was found to be almost completely replaced by the regenerated myofibers (Fig. 7B and C).

30 days after virus vector administration, the size of muscle returned nearly to normal and evenly in the treated muscles of the group 1 animals (Fig. 8 and 9). In the hIGF-I/SeV treated muscles of the group 1 animals, cluster of medium sized myofibers was interspersed among the normal sized myofibers (data not shown). The total number of myofibers in the hIGF-I/SeV treated muscle of the group 1 animals was 17% higher than that in the LacZ/SeV treated muscle (P<0.03, n=4) (Fig. 9). In groups 3 and 4, no statistical significant difference in the numbers of fibers existed between the right and left muscles (data not shown). Furthermore, the numbers of splitting fibers, which is a common result of hypertrophy, increased. The average number of splitting fibers in hIGF-I/SeV treated muscles of group 1 animals was 6.1 times as much as those in LacZ/SeV treated muscles (Fig. 9). These data indicate that this splitting phenomenon might have induced the increase in the total number of myofibers in muscles treated with hIGF-I/SeV.

The effect of IGF-I on mature skeletal muscles have been reported several times. Adans and McCue (J. Appl. Physiol. 84 (5), 1716-1722 (1998)) investigated the effect of local infusion of IGF-I protein into normal rat tibialis anterior muscle. A significant increase in muscle weight, total protein, and total DNA could be observed in the IGF-I injected muscle. However, they failed to report the increase in the total number of myofibers. Barton-Davis *et al*. (Proc. Natl. Acad. Sci. USA 95, 15603-15607 (1998)) transferred the IGF-I gene into aged rats using an AAV vector. Although fiber regeneration and muscle hypertrophy could be observed, no increase in the number of regenerated myofiber could be detected. In the study by the present inventors, a significant increase in the number of regenerated fibers, hypertrophied fibers, and total fibers was observed in normal adult muscle due to the effect of overexpressed hIGF-I. The observed new myofiber formation may be a result of the high expression level of IGF-I achieved by the SeV vector.

### [Example 5] Comparison of Gene Expression of Purified LacZ-SeV/dF and LacZ-SeV in mouse leg muscle

Gene expression of additive Sendai virus vector derived from developing chicken egg chorio-allantoic fluid (LacZ-SeV) and purified F-deficient type LacZ Sendai virus vector derived from LLC/MK2/F/Ad cells (LacZ-SeV/dF) injected into leg muscle (dosage: 4 x 10⁷ CIU/head) was compared by intramuscular administration using BALB/c mice.

18 six-week-old male BALB/c mice (Charles River Japan, Inc.) were used in the experiment. The animals were acquired at the age of six weeks, and after a two-day acclimation period, the animals were divided into three groups each consisting of six animals: (1) untreated group; (2) LacZ-SeV (4 x 10⁷ CIU/head) group; and (3) LacZ-SeV/dF (4 x 10⁷ CIU/head) group. Each viral solution was administered under ether anesthesia at a dosage of 2 x 10⁸ CIU/ml, 100 µl per cite at two cites for each animal on both sides of the tibialis anterior muscle using a 29G syringe while immobilizing the legs of the animals. The muscles were rubbed for about 1 minute after the administration.

Three days after the administration, the animals were anatomized under ether anesthesia, and the leg muscle on the administered (right) side were sampled and used for LacZ assay.

Quantification of LacZ was carried out as described below. First, leg muscle was excised (2.0 ml microtube) and frozen with liquid nitrogen. Next, 500 µl of Lysis solution was added (muscle weight: approx. 500 mg), and the muscle was homogenized (MultiPro) while cooling on ice. The homogenate was centrifuged (15000 rpm x 15 min), and 10 µl of supernatant was sampled into an assay tube. Then, 70 µl of Reaction Buffer A (100 µl kit containing 1 µl of Galacton-Plus and 99 µl of Reaction Buffer; Galacton-Light-Plus, TROPIX, Cat. No. 250065) was added and left standing for 30-60 minutes at room temperature while blocking from light. 100 µl of Accelerator was added to measure with luminometer.

By comparing the gene expression (quantitative) in leg muscle 3 days after administration, LacZ-SeV/dF and LacZ-SeV clearly exhibited stronger expression as compared with the untreated group (Fig. 10). When LacZ-SeV/dF and LacZ-SeV were compared, although there was some variation, the expression was determined to be nearly equal. This is believed to be due to the low influence of secondary infection in the muscle. For the treatment of muscle diseases by gene therapy, equal effects can be expected by the use of F-deficient type and the additive type viruses since no differences in gene expression were detected between the two.

### Industrial Applicability

The present invention enabled introduction of genes into skeletal muscle with extremely high efficiency using Paramyxovirus vector. As a result, the present invention provides a basic technique for gene therapy targeting skeletal muscle, particularly for systemic diseases and neuromuscular disorders. According to another aspect of the present invention, introduction into skeletal muscle of a gene encoding an insulin-like growth factor using the Paramyxovirus vector of the present invention is expected to be applicable for the treatment of atrophy, reduction, and denaturation of myofibers. These findings are expected to potentiate current treatment methods for neuromuscular disorders.

## Claims

1. A method for introducing a foreign gene into skeletal muscle, wherein said method comprises the step of administering a Paramyxovirus vector inserted with the foreign gene into skeletal muscle.

2. The method according to claim 1, wherein the Paramyxovirus is Sendai virus.

3. The method according to claim 1 or 2, wherein the foreign gene is a therapeutic gene.

4. The method according to claim 1 or 2, wherein the foreign gene is a gene that encodes an insulin-like growth factor.

5. A Paramyxovirus vector inserted with a foreign gene which is used for introducing the foreign gene into skeletal muscle.

6. The vector according to claim 5, wherein the Paramyxovirus is Sendai virus.

7. The vector according to claim 5 or 6, wherein the foreign gene is a therapeutic gene.

8. The vector according to claim 5 or 6, wherein the foreign gene encodes an insulin-like growth factor.

9. A composition for introducing a foreign gene into skeletal muscle which comprises a Paramyxovirus vector inserted with the foreign gene.

10. The composition according to claim 9, wherein the Paramyxovirus is Sendai virus.

11. The composition according to claim 9 or 10, wherein the foreign gene is a therapeutic gene.

12. The composition according to claim 9 or 10, wherein the foreign gene encodes an insulin-like growth factor.

13. The composition according to claim 12 which is used for increasing regenerating myofibers and/or splitting myofibers in mammal.

14. The composition according to claim 12 which is used for the treatment of neuromuscular disorders.


SEQUENCE LISTING

<110> DNAVEC Research Inc.

<120> PARAMYXOVIRUS VECTORS FOR INTRODUCING FOREIGN GENES INTO SKELETAL
MUSCLE

<130> D3-A0007P

<140>
<141>

<150> JP 2000-308533
<151> 2000-10-06

<160> 4

<170> PatentIn Ver. 2.0

<210> 1
<211> 32
<212> DNA
<213> Artificial Sequence

<220>

<223> Description of Artificial Sequence:Artificially

Synthesized Primer Sequence.

<400> 1

atccgaattc gcaatgggaa aaatcagcag tc 32

<210> 2

<211> 37

<212> DNA

<213> Artificial Sequence

<220>

<223> Description of Artificial Sequence:Artificially

Synthesized Primer Sequence.

<400> 2

atccgaattc ctacatcctg tagttcttgt ttcctgc 37

<210> 3

<211> 46

<212> DNA

<213> Artificial Sequence

<220>

<223> Description of Artificial Sequence:Artificially

Synthesized Primer Sequence.

<400> 3

atccgcggcc gccaaagttc agcaatggga aaaatcagca gtcttc                46

<210> 4

<211> 73

<212> DNA

<213> Artificial Sequence

<220>

<223> Description of Artificial Sequence:Artificially

      Synthesized Primer Sequence.

<400> 4

atccgcggcc gcgatgaact ttcaccctaa gtttttctta ctacggctac atcctgtagt 60

tcttgtttcc tgc                                                    73